# EUROPÄISCHE PATENTSCHRIFT

(11) **EP 3 164 695 B1**
(45) Veröffentlichungstag und Bekanntmachung des Hinweises auf die Patenterteilung: **05.06.2019**
(21) Anmeldenummer: 15734626.3
(22) Anmeldetag: 30.06.2015
(51) Int. Cl.: G01N 21/55, G01N 33/20, G06T 7/00, G01B 11/30, B23K 26/03, B23K 26/14, B23K 26/24, B23K 26/32, B23K 26/38, B23K 26/40, B23K 26/70, B23K 26/60, G01N 21/86, G01N 21/84, G01N 21/89

(54) **VERFAHREN UND VORRICHTUNG ZUM BESTIMMEN EINER WERKSTOFFART UND/ODER EINER OBERFLÄCHENBESCHAFFENHEIT EINES WERKSTÜCKS**
METHOD AND DEVICE FOR DETERMINING A MATERIAL TYPE AND/OR SURFACE CHARACTERISTICS OF A WORKPIECE
PROCÉDÉ ET DISPOSITIF DE DÉTERMINATION D'UN TYPE DE MATÉRIAU ET/OU D'UNE PROPRIÉTÉ DE SURFACE D'UNE PIÈCE

(30) Priorität: 01.07.2014 DE 102014212682
(43) Veröffentlichungstag der Anmeldung: 10.05.2017
(73) Patentinhaber: Trumpf Werkzeugmaschinen GmbH + Co. KG, 71254 Ditzingen (DE)
(72) Erfinder: HALLASCH, Dieter, 71254 Ditzingen (DE); HESSE, Tim, 71254 Ditzingen (DE); REGAARD, Boris, 70499 Stuttgart (DE); SCHINDHELM, David, 70176 Stuttgart (DE)
(74) Vertreter: Kohler Schmid Möbus Patentanwälte
(86) Internationale Anmeldenummer: PCT/EP2015/064864
(87) Internationale Veröffentlichungsnummer: WO 2016/001234

(56) Entgegenhaltungen:
- WO-A1-2010/057661
- DE-A1- 10 324 104
- DE-A1- 10 339 227
- DE-A1- 19 846 619
- US-A- 5 155 558
- JUPTNER W ET AL: "OPTO-ELEKTRONISCHER SENSOR FUER DIE ECHTZEITBEOBACHTUNG BEIM LASERSCHWEISSEN ZUR NAHTFUEHRUNG UND ADAPTIVEN PROZESSBEEINFLUSSUNG", LASER UND OPTOELEKTRONIK, FACHVERLAG GMBH. STUTTGART, DE, Bd. 22, Nr. 6, 1. Oktober 1990 (1990-10-01), Seiten 56-62, XP000175758, ISSN: 0722-9003

## Beschreibung

Die vorliegende Erfindung betrifft ein Verfahren und eine Vorrichtung zum Bestimmen einer Werkstoffart und/oder einer Oberflächenbeschaffenheit eines bevorzugt metallischen, insbesondere plattenförmigen Werkstücks, sowie ein Computerprogrammprodukt zur Durchführung des Verfahrens.

Bei der Bearbeitung von Werkstücken an einer Werkzeugmaschine, beispielsweise beim Schneiden von Werkstücken mittels eines Hochenergiestrahls, ist es typischer Weise erforderlich, die Werkstoffart (z.B. Baustahl, Edelstähle, Buntmetalle, ...) sowie die Oberflächenbeschaffenheit (z.B. Oxidation der Oberfläche, Rauheit, Beschichtung der Oberfläche z.B. mit Farbe oder Folie, ...) des Werkstücks zu kennen, um Bearbeitungsparameter wie z.B. Vorschub, Laserleistung, Gasart und Gasdruck geeignet anzupassen. Die Werkstoffart bzw. die Oberflächenbeschaffenheit eines zu bearbeitenden Werkstücks wird typischer Weise manuell von einem Bediener der Werkzeugmaschine vorgegeben. Gibt der Bediener an der Werkzeugmaschine eine Werkstoffart bzw. eine Oberflächenbeschaffenheit vor, die nicht mit der tatsächlich zu bearbeitenden Werkstoffart bzw. Oberflächenbeschaffenheit des zu bearbeitenden Werkstücks übereinstimmt, ist typischer Weise ein minderwertiges Ergebnis des Bearbeitungsprozesses die Folge. Aus der DE 10 2011 005 907 B3 ist ein Verfahren zum Bestimmen der Reflektivität einer Oberfläche bekannt geworden, die mit einer Auflichtbeleuchtung beleuchtet wird, wobei von der Oberfläche Bilder mit unterschiedlichen Beleuchtungszeiten aufgenommen werden und in jedem der aufgenommenen Bilder derjenige helle Flächenanteil bestimmt wird, in dem eine vorbestimmte Mindesthelligkeit erreicht ist. Aus dem bestimmten hellen Flächenanteil wird in Abhängigkeit von den unterschiedlichen Belichtungszeiten die Änderung des hellen Flächenanteils mit der Belichtungszeit und hieraus die Reflektivität der Oberfläche bestimmt, um hochreflektierende und matte Oberflächen zu erkennen und zu unterscheiden.

Aus der DE 103 24 104 A1 ist ein Verfahren Verfahren zur Bestimmung von Oberflächeneigenschaften bekannt geworden, wobei in einem ersten Verfahrensschritt eine vorgegebene Strahlung von wenigstens einer Strahlungseinrichtung auf eine Messfläche gesandt wird, in weiteren Verfahrensschritten, die von der Messfläche gestreute und/oder reflektierte Strahlung mittels einer Vielzahl von Bildaufnahmeelementen detektiert wird, ein Signal erzeugt wird, das wenigstens einen Parameter der von den Bildaufnahmeelementen detektierten Strahlung beschreibt. In weiteren Verfahrensschritten werden die ersten Signale gemäß vorgegebenen Kriterien zu Gruppensignalen zusammengefasst sowie wenigstens eine gruppenspezifische Bewertungszahl sowie ein davon abhängiger statistischer Parameter ermittelt, der mit wenigstens einer Remissionseigenschaft der Messfläche korreliert. Schließlich wird wenigstens ein statistischer Parameter in Abhängigkeit des vorgegebenen Kriteriums zum Zusammenfassen der ersten Signale ausgegeben. Dabei wird die Oberflächeneigenschaft durch eine Relation zwischen wenigstens zwei statistischen Parametern mit unterschiedlichen Kriterien bestimmt.

### Aufgabe der Erfindung

Der Erfindung liegt die Aufgabe zugrunde, ein Verfahren und eine Vorrichtung bereitzustellen, welche eine automatisierte Bestimmung der Werkstoffart und/oder der Oberflächenbeschaffenheit eines Werkstücks ermöglichen.

### Gegenstand der Erfindung

Diese Aufgabe wird erfindungsgemäß durch ein Verfahren der eingangs genannten Art gelöst, welches folgende Schritte umfasst: Beleuchten einer Oberfläche des Werkstücks mit Beleuchtungsstrahlung, Aufnehmen mindestens eines Bildes der beleuchteten Oberfläche, sowie Bestimmen der Werkstoffart und/oder der Oberflächenbeschaffenheit des Werkstücks anhand einer statistischen Analyse des mindestens einen in den Orts-Frequenzraum gewandelten Bildes, wobei die Werkstoffart und/oder die Oberflächenbeschaffenheit des Werkstücks anhand einer Anisotropie der Häufigkeits-Verteilung der Orts-Frequenzen und/oder anhand mindestens einer richtungsunabhängigen Eigenschaft der Häufigkeits-Verteilung der Orts-Frequenzen des in den Orts-Frequenzraum gewandelten Bildes bestimmt werden. Bei der Beleuchtung der Oberfläche des Werkstücks handelt es sich typischer Weise um eine Hellfeld-Auflichtbeleuchtung, bei welcher die Beleuchtungsstrahlung direkt von der Oberfläche des Werkstücks in die Beobachtungsrichtung zurück reflektiert wird.

Erfindungsgemäß wird vorgeschlagen, eine automatisierte Klassifikation von unterschiedlichen Werkstoffarten bzw. Materialien und/oder unterschiedlichen Oberflächenbeschaffenheiten des Werkstücks vorzunehmen, um - in der Regel ebenfalls automatisiert - Bearbeitungsparameter und/oder Bearbeitungstechnologien auszuwählen, die für die Bearbeitung des Werkstücks in der Werkzeugmaschine optimiert bzw. besonders gut geeignet sind. Die Bestimmung der Werkstoffart und/oder der Oberflächenbeschaffenheit des Werkstücks erfolgt anhand der Analyse des mindestens einen aufgenommenen Bildes der Oberfläche des Werkstücks, welches in den Orts-Frequenzraum gewandelt wurde. Im Orts-Frequenzraum können charakteristische Strukturen der Oberfläche des Werkstücks, insbesondere Hell-Dunkel-Strukturen besonders gut erkannt bzw. analysiert werden. Es hat sich insbesondere gezeigt, dass der Orts-Frequenzraum des aufgenommenen Bildes einer gegebenen Oberfläche sich signifikant vom Orts-Frequenzraum anderer Oberflächen unterscheidet, so dass eine Unterscheidung unterschiedlicher Werkstoffarten und/oder Oberflächenbeschaffenheiten anhand eines in den Orts-Frequenzraum gewandelten Bildes der Oberfläche des Werkstücks besonders vorteilhaft ist.

Erfindungsgemäß werden die Werkstoffart und/oder die Oberflächenbeschaffenheit des Werkstücks anhand einer Anisotropie der Häufigkeits-Verteilung der Orts-Frequenzen und/oder anhand mindestens einer richtungsunabhängigen Eigenschaft (typischer Weise einem richtungsunabhängigen Streuungsmaß) der Häufigkeits-Verteilung der Orts-Frequenzen des in den Orts-Frequenzraum gewandelten Bildes bestimmt. Die statistische Analyse der Häufigkeits-Verteilung der Orts-Frequenzen des in den Orts-Frequenzraum gewandelten Bildes kann mit Hilfe von bekannten Methoden aus der Bildverarbeitung, beispielsweise durch Berechnung der zweidimensionalen örtlichen Momente (engl. "spatial moments") erfolgen, bei der die Elliptizität der örtlichen Frequenzverteilung berechnet wird, welche ein Maß für die Anisotropie der Häufigkeitsverteilung der Orts-Frequenzen in der Oberflächenstruktur darstellt, wobei die örtlichen Momente den maximalen und minimalen Radius der umschließenden Ellipse der Standard-Abweichung, d.h. die Hauptachsen der Standard-Abweichung der Häufigkeits-Verteilung der Orts-Frequenzen liefern. Als richtungsunabhängige Eigenschaft der Häufigkeits-Verteilung der Frequenzen im Orts-Frequenzraum kann beispielsweise ein skalares Streuungsmaß, beispielsweise die (skalare) Standardabweichung oder die Varianz der Häufigkeits-Verteilung dienen. Sowohl die richtungsunabhängige Eigenschaft auch die Anisotropie im Orts-Frequenzraum, d.h. der richtungsabhängige Orts-Frequenzraum, der beispielsweise durch die charakteristischen Größen maximaler Radius und minimaler Radius der umschließenden Ellipse ("spatial moments") der Häufigkeits-Verteilung beschrieben werden kann, können als Unterscheidungskriterien zwischen unterschiedlichen Werkstückarten bzw. unterschiedlichen Oberflächenbeschaffenheiten verwendet werden. Insbesondere können diese beiden Unterscheidungskriterien zusammen mit dem Reflexionsgrad als drittem Unterscheidungskriterium zur Bestimmung der Werkstoffart und/oder zur Bestimmung der Oberflächenbeschaffenheit genutzt werden.

Zusätzlich zur Bestimmung der Werkstoffart und/oder der Oberflächenbeschaffenheit anhand des in den Orts-Frequenzraum gewandelten Bildes ist es auch möglich, für die Bestimmung der Werkstoffart bzw. der Oberflächenbeschaffenheit des Werkstücks den Reflexionsgrad der Oberfläche des Werkstücks für die Beleuchtungsstrahlung zu berücksichtigen. Der Reflexionsgrad kann hierbei für eine Wellenlänge, beispielsweise für die Wellenlänge der Beleuchtungsstrahlung, d.h. als absoluter Reflexionsgrad bestimmt werden. Es ist aber auch möglich, den Reflexionsgrad der Oberfläche des Werkstücks für mehrere unterschiedliche Wellenlängen der Beleuchtungsstrahlung zu bestimmen und die jeweiligen Reflexionsgrade miteinander in Beziehung zu setzen (relative Reflexion), um die Oberflächenbeschaffenheit und/oder die Werkstoffart zu bestimmen. Es kann auch der maximale, minimale und mittlere Reflexionsgrad der Oberfläche bestimmt werden. Um unterschiedliche Werkstoffarten und/oder Oberflächenbeschaffenheiten zu unterscheiden, kann zusätzlich zu den aus der Analyse des in den Orts-Frequenzraum gewandelten Bildes gewonnenen Unterscheidungskriterien der (absolute oder relative) Reflexionsgrad als weiteres Unterscheidungskriterium verwendet werden.

Bei einer Weiterbildung werden zum Bestimmen der Werkstoffart und/oder der Oberflächenbeschaffenheit des Werkstücks der Reflexionsgrad, die Anisotropie der Orts-Frequenzen und/oder die Häufigkeits-Verteilung der Orts-Frequenzen mit Referenzdaten für unterschiedliche Werkstoffarten und/oder Oberflächenbeschaffenheiten verglichen. Die Referenzdaten für den Reflexionsgrad, die Anisotropie der Orts-Frequenzen und/oder der Häufigkeits-Verteilung der Orts-Frequenzen für unterschiedliche Werkstoffarten und/oder Oberflächenbeschaffenheiten können in einer Datenbank hinterlegt sein. Anhand des Vergleichs der bei der Analyse des Bildes ermittelten Werte der obigen Unterscheidungskriterien mit jeweiligen Referenzdaten kann diejenige Werkstoffart bzw. Oberflächenbeschaffenheit bestimmt werden, die den bei der Analyse des Bildes ermittelten Werten am Nächsten kommt.

Bei einer Weiterbildung erfolgt das Vergleichen mittels eines lernenden Systems, insbesondere mittels eines (künstlichen) Neuronalen Netzes Ein lernendes System kann während einer Lernphase mit Referenzdaten für die Anisotropie der Orts-Frequenzen, die Häufigkeits-Verteilung der Orts-Frequenzen und/oder den Reflexionsgrad unterschiedlicher bekannter Werkstoffarten und/oder Oberflächenbeschaffenheiten trainiert werden. Werden die bei der Analyse des Bildes des Werkstücks ermittelten Werte für die Anisotropie, die Häufigkeits-Verteilung und/oder den Reflexionsgrad einem solchen trainierten Neuronalen Netzwerk als Eingangsgrößen zur Verfügung gestellt, ordnet das Neuronale Netzwerk diesen Eingangsgrößen automatisch diejenige Werkstoffart und/oder Oberflächenbeschaffenheit zu, die den gegebenen Eingangsgrößen am nächsten kommen. An Stelle eines lernenden Systems können andere Verfahren für den Vergleich verwendet werden, beispielsweise das so genannte "Template Matching", bei dem kleine Teilbereiche eines Bildes mit vorgegebenen Bildbestandteilen ("Templates") verglichen werden, SAD ("sum of absolute difference"), etc.

Bei einer Variante wird die Werkstoffart bestimmt aus einer Gruppe, die Baustahl, Edelstahl und Buntmetalle umfasst, d.h. das Werkstück wird als Baustahl, Edelstahl oder als Buntmetall klassifiziert. Die Klassifizierung der Werkstoffart muss sich nicht zwingend auf die Einteilung in eine der drei oben angegebenen Arten von Werkstücken beschränken. Vielmehr können ggf. unterschiedliche Arten von Baustählen unterschieden werden und/oder es kann in der Materialklasse der Buntmetalle eine Bestimmung eines jeweiligen Buntmetalls, z.B. Kupfer, Aluminium etc. erfolgen.

Bei einer weiteren Variante umfasst das Bestimmen der Oberflächenbeschaffenheit das Erkennen einer gewalzten Oberfläche des Werkstücks. Da gewalzte Bleche eine von der Walzrichtung abhängige Riefenstruktur aufweisen, kann anhand der Anisotropie der Orts-Frequenzen im in den Orts-Frequenzraum umgewandelten bzw. transformierten Bild erkannt werden, ob es sich um ein gewalztes Werkstück handelt. Insbesondere kann hierbei ein gewalztes von einem z.B. im Druckgussverfahren hergestellten oder mit Farbe, Folie oder dergleichen beschichteten Werkstück unterschieden werden, da derartige Werkstücke in der Regel keine im Orts-Frequenzraum als Anisotropie zu erkennende Vorzugsrichtung aufweisen. Zusätzlich oder alternativ zur Walzrichtung des Werkstücks können auch andere Oberflächeneigenschaften, z.B. die Rauheit oder die Oxidation der Oberfläche, bestimmt werden.

Bei einer weiteren Variante werden beim Aufnehmen des Bildes eine Belichtungszeit und/oder eine Beleuchtungsintensität an den Reflexionsgrad der Oberfläche des Werkstücks angepasst. Die Beleuchtungsintensität bzw. die Belichtungszeit werden hierbei so gewählt, dass das aufgenommene Bild einen möglichst hohen Kontrast aufweist. Anhand der für die Erzeugung eines hohen Kontrasts erforderlichen Belichtungszeit bzw. Beleuchtungsintensität kann indirekt auf den Reflexionsgrad der Oberfläche geschlossen werden, d.h. es ist nicht erforderlich, den Reflexionsgrad der Oberfläche mittels einer separaten Reflektivitätsmessung zu bestimmen.

Bei einer weiteren Variante umfasst das Bestimmen der Werkstoffart und/oder der Oberflächenbeschaffenheit das Bilden eines Mittelwerts aus einer Zahl zwischen 10 und 1000 in den Orts-Frequenzraum gewandelter Bilder. Die Bestimmung der Werkstoffart und/oder der Oberflächenbeschaffenheit des Werkstücks anhand von gemittelten Bildsequenzen ist gegenüber der Bestimmung anhand eines einzigen Bildes in der Regel deutlich verbessert. Die Analyse des Orts-Frequenzbereichs eines einzelnen Bildes ermöglicht häufig bereits eine eindeutige Unterscheidung unterschiedlicher Materialarten bzw. Oberflächenbeschaffenheiten.

In einer weiteren Variante erfolgt das Beleuchten der Oberfläche des Werkstücks mit Laserstrahlung als Beleuchtungsstrahlung, d.h. mit Beleuchtungsstrahlung, die (mindestens) eine diskrete Wellenlänge aufweist. Bei der Beleuchtungsquelle der Beleuchtungseinrichtung kann es sich beispielsweise um einen Diodenlaser handeln, der Beleuchtungsstrahlung bei einer Wellenlänge von beispielsweise 660nm, 808nm, 915nm, 980nm nm erzeugt. Das Beleuchten der Oberfläche sollte möglichst homogen erfolgen. Die Beleuchtungsstrahlung kann beispielsweise über eine Faser oder einen Glasstab homogenisiert werden. Die Beleuchtung der Oberfläche erfolgt bevorzugt als Auflichtbeleuchtung, da bei der Auflichtbeleuchtung durch die natürliche Rauigkeit der Oberfläche des Werkstücks ein hoher Kontrast erzeugt wird. Beleuchtungsstrahlung mit unterschiedlichen Wellenlängen kann dazu verwendet werden, um den Reflexionsgrad bzw. die Reflexionsintensität der Oberfläche bei unterschiedlichen Wellenlängen zu bestimmen und die Reflexionsintensitäten zu vergleichen. Wie weiter oben beschrieben wurde, kann der Reflexionsgrad bei unterschiedlichen Wellenlängen als weiteres Unterscheidungskriterium zwischen unterschiedlichen Werkstoffarten und/oder Oberflächenbeschaffenheiten dienen.

Bei einer vorteilhaften Weiterbildung wird die Beleuchtungsstrahlung koaxial zu einem Hochenergiestrahl zur Bearbeitung des Werkstücks auf die Oberfläche eingestrahlt. Bei der Bearbeitung des Werkstücks mit einem Hochenergiestrahl, beispielsweise mit einem Laserstrahl, z.B. beim Laserstrahlschneiden oder beim Laserstrahlschweißen, oder mit einem Plasmastrahl, hat es sich als günstig erwiesen, wenn die Beleuchtung der Oberfläche des Werkstücks und/oder die Beobachtung der Oberfläche des Werkstücks koaxial zum Hochenergiestrahl erfolgt. Auf diese Weise wird eine minimale Störkontur erzeugt und die Bestimmung der Werkstoffart und/oder der Oberflächenbeschaffenheit kann sowohl unmittelbar vor der Bearbeitung als auch während der Bearbeitung (d.h. hauptzeitparallel) erfolgen. Die Einkopplung der Beleuchtungsstrahlung in sowie die Auskopplung des Beobachtungsstrahlengangs aus dem Strahlengang des Hochenergiestrahls kann beispielsweise durch eine örtliche (z.B. seitliche) Ein- bzw. Auskopplung oder durch einen teildurchlässigen Spiegel (Dichroit) erfolgen.

Bei einer weiteren Variante umfasst das Verfahren zusätzlich: Festlegen mindestens eines Bearbeitungsparameters zum Bearbeiten des Werkstücks abhängig von der Werkstoffart und/oder der Oberflächenbeschaffenheit. Wie weiter oben beschrieben wurde, können anhand der auf die oben beschriebene Weise bestimmten Werkstoffart und/oder der Oberflächenbeschaffenheit die Bearbeitungsparameter des Bearbeitungsprozesses, z.B. eines Laserschweißprozesses oder eines Laserschneidprozesses, wie z.B. die Vorschubgeschwindigkeit, die Laserleistung, die Art des dem Werkstück als Hilfsgas oder als Schneidgas zugeführten Gases sowie der Gasdruck eines solchen Gases optimiert werden. Abhängig von der Werkstoffart und/oder von der Oberflächenbeschaffenheit des Werkstücks kann auch eine Auswahl von geeigneten Bearbeitungstechnologien zur Bearbeitung des Werkstücks, beispielsweise die Auswahl eines für die Bearbeitung geeigneten Lasers bzw. einer geeigneten Laserwellenlänge, erfolgen.

Die Erfindung betrifft auch ein Computerprogrammprodukt, welches zur Durchführung aller Schritte des oben beschriebenen Verfahrens ausgebildet ist, wenn das Computerprogramm auf einer Datenverarbeitungsanlage abläuft. Bei der Datenverarbeitungsanlage kann es sich beispielsweise um eine Steuereinrichtung, eine Regeleinrichtung oder um eine Auswerteeinrichtung handeln, die Teil einer Bearbeitungsmaschine für das Werkstück ist. Es kann sich aber auch um eine externe Einrichtung, beispielsweise einen PC, handeln, welche mit der Bearbeitungsmaschine zum Austausch von Daten in Verbindung steht.

Ein weiterer Aspekt der Erfindung betrifft eine Vorrichtung der eingangs genannten Art, umfassend alle im Anspruch 11 angegebenen Merkmale, unter anderem: eine Beleuchtungseinrichtung zur Erzeugung von Beleuchtungsstrahlung zum Beleuchten einer Oberfläche des Werkstücks, eine Bilderfassungseinrichtung zur Aufnahme mindestens eines Bildes der beleuchteten Oberfläche des Werkstücks, sowie eine Auswerteeinrichtung, die zur Bestimmung der Werkstoffart und/oder der Oberflächenbeschaffenheit des Werkstücks anhand des mindestens einen in den Orts-Frequenzraum gewandelten Bildes ausgebildet bzw. programmiert ist.

Wie weiter oben im Zusammenhang mit dem Verfahren beschrieben wurde, kann mit Hilfe der Vorrichtung eine automatisierte Bestimmung der Werkstoffart und/oder der Oberflächenbeschaffenheit des Werkstücks erfolgen. Die Vorrichtung kann beispielsweise als Bearbeitungskopf, insbesondere als Laserbearbeitungskopf, ausgebildet sein. In diesem Fall ist die Auswerteeinrichtung typischer Weise in den Bearbeitungskopf integriert. Bei der Vorrichtung kann es sich auch um eine Bearbeitungsmaschine, insbesondere um eine Laserbearbeitungsmaschine, handeln. In diesem Fall ist die Auswerteeinrichtung typischer Weise vom Bearbeitungskopf separiert angeordnet.

Bei einer Ausführungsform ist die Bilderfassungseinrichtung zur Aufnahme des mindestens einen Bildes eines durch eine Fokussierlinse zum Fokussieren eines Hochenergiestrahls auf das Werkstück verlaufenden Beobachtungsstrahlengangs ausgebildet. Die Bilderfassungseinrichtung ist derart angeordnet bzw. ausgerichtet, dass der Beobachtungsstrahlengang durch die Fokussierlinse verläuft, durch die auch ein Hochenergiestrahl, beispielsweise ein Laserstrahl oder ein Plasmastrahl, hindurch tritt, der zur Bearbeitung eines Werkstücks verwendet wird. Für die Realisierung eines solchen Beobachtungsstrahlengangs kann der Bilderfassungseinrichtung eine Abbildungsoptik mit einem oder mehreren geeignet positionierten optischen Elementen, z.B. in Form einer oder mehrerer Linsen, zugeordnet sein.

Bei einer Weiterbildung ist die Bilderfassungseinrichtung ausgebildet, das mindestens eine Bild aus einer Beobachtungsrichtung koaxial zur Hauptachse der Fokussierlinse aufzunehmen. Die Strahlachse des Hochenergiestrahls stimmt im Idealfall mit der Hauptachse der Fokussierlinse überein, die zentrisch durch die Fokussierlinse verläuft, d.h. der Hochenergiestrahl tritt zentrisch durch die Fokussierlinse hindurch. Durch die koaxiale Beobachtung der typischer Weise senkrecht zur Hauptachse der Fokussierlinse ausgerichteten planen Werkstückoberfläche kann eine richtungsunabhängige Beobachtung realisiert werden, was für die Bestimmung der Werkstoffart und/oder der Oberflächenbeschaffenheit des Werkstücks günstig ist.

Bei einer Weiterbildung ist die Beleuchtungseinrichtung zur Beleuchtung der Oberfläche des Werkstücks durch die Fokussierlinse, bevorzugt koaxial zur Hauptachse der Fokussierlinse, ausgebildet. Wie weiter oben beschrieben wurde, ist eine Auflichtbeleuchtung der Oberfläche des Werkstücks günstig, da hierbei aufgrund der natürlichen Rauigkeit der Werkstückoberfläche ein hoher Kontrast erzeugt wird.

Bei einer weiteren Ausführungsform umfasst die Beleuchtungseinrichtung als Beleuchtungsquelle mindestens einen Laser, insbesondere einen Diodenlaser, oder eine Leuchtdiode. Für die Bestimmung der Werkstückart und/oder der Oberflächenbeschaffenheit des Werkstücks hat es sich als günstig erwiesen, wenn die Beleuchtung bei mindestens einer diskreten Wellenlänge sowie mit einer homogenen Beleuchtungsquelle mit hoher Strahlqualität, beispielsweise mit einem Diodenlaser, erfolgt. Die Einkopplung der Beleuchtungsstrahlung in den Strahlengang des Hochenergiestrahls kann beispielsweise seitlich oder mittels eines teiltransmissiven Spiegels erfolgen.

Erfindungsgemäß ist die Auswerteeinrichtung ausgebildet bzw. programmiert, die Werkstoffart und/oder die Oberflächenbeschaffenheit des Werkstücks anhand einer Anisotropie der Häufigkeits-Verteilung der Orts-Frequenzen und/oder mindestens einer richtungsunabhängigen Eigenschaft der Häufigkeits-Verteilung der Orts-Frequenzen des in den Orts-Frequenzraum gewandelten Bildes zu bestimmen. Wie weiter oben im Zusammenhang mit dem Verfahren beschrieben wurde, kann der richtungsabhängige örtliche Frequenzraum bzw. die Anisotropie der Orts-Frequenzen sowie die mindestens eine richtungsunabhängige Eigenschaft der Häufigkeits-Verteilung der Orts-Frequenzen mit Hilfe von bekannten Methoden der Bildverarbeitung berechnet werden, beispielsweise durch die Berechnung der örtlichen Momente.

Bei einer weiteren Ausführungsform ist die Auswerteeinrichtung ausgebildet, zum Bestimmen der Werkstoffart und/oder der Oberflächenbeschaffenheit des Werkstücks zusätzlich der Reflexionsgrad der Oberfläche des Werkstücks zu berücksichtigen. Wie weiter oben beschrieben wurde, kann der Reflexionsgrad der Oberfläche, ggf. gemessen für unterschiedliche Wellenlängen der Beleuchtungsstrahlung, als zusätzliches Unterscheidungskriterium dienen, um unterschiedliche Werkstoffarten und/oder Oberflächenbeschaffenheiten unterscheiden zu können.

In einer Weiterbildung ist die Auswerteeinrichtung ausgebildet bzw. programmiert, zum Bestimmen der Werkstoffart und/oder der Oberflächenbeschaffenheit den Reflexionsgrad, die Anisotropie der Orts-Frequenzen und/oder die Häufigkeits-Verteilung der Orts-Frequenzen mit Referenzdaten für unterschiedliche Werkstoffarten und/oder Oberflächenbeschaffenheiten zu vergleichen. Die Referenzdaten für den Reflexionsgrad, die Anisotropie der Orts-Frequenzen und/oder der Häufigkeits-Verteilung der Orts-Frequenzen für unterschiedliche Werkstoffarten und/oder Oberflächenbeschaffenheiten können in einer Datenbank hinterlegt sein. Anhand des Vergleichs der drei Unterscheidungskriterien mit den jeweiligen Referenzdaten kann diejenige Werkstoffart bzw. Oberflächenbeschaffenheit bestimmt werden, die den bei der Analyse des Bildes ermittelten Werten am Nächsten kommt. Die Auswerteeinrichtung kann insbesondere ausgebildet sein, das Vergleichen mittels eines selbstlernenden Systems, beispielsweise mittels eines (künstlichen) Neuronalen Netzes, durchzuführen. Es ist aber auch möglich, den Vergleich bzw. die Suche ähnlicher Parameter in der Datenbank mit Hilfe einer herkömmlichen Minimierungsfunktion durchzuführen, beispielsweise durch die Minimierung der kleinsten Fehlerquadrate.

Weitere Vorteile der Erfindung ergeben sich aus der Beschreibung und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigen:
- Fig. 1: eine schematische Darstellung eines Ausführungsbeispiels einer Laserbearbeitungsmaschine zur Bestimmung einer Werkstoffart und/oder einer Werkstoffbeschaffenheit eines zu bearbeitenden Werkstücks,
- Fig. 2a-c: Darstellungen von drei Bildern im Ortsraum sowie von drei in den Orts-Frequenzraum gewandelten Bildern einer Werkstückoberfläche eines plattenförmigen Werkstücks, sowie
- Fig. 3a-d: Darstellungen von vier Bildern von Werkstückoberflächen von Werkstücken unterschiedlicher Baustahlsorten.

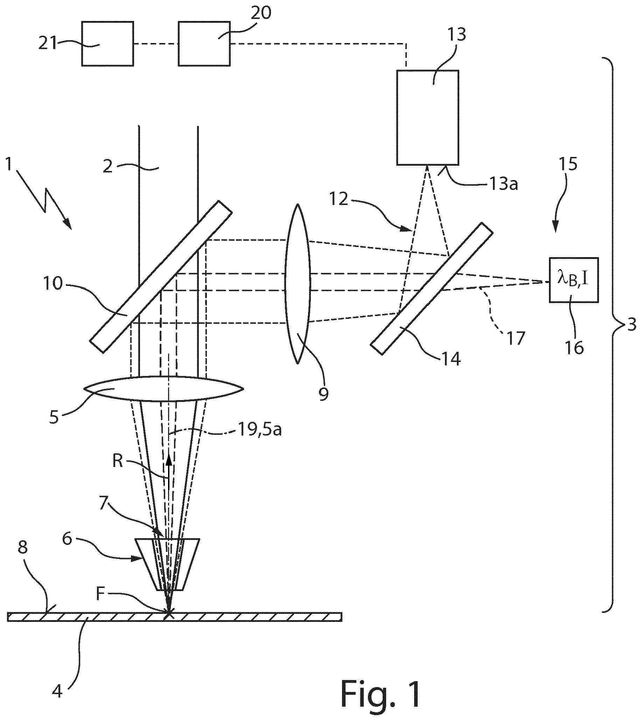

**Fig. 1** zeigt einen beispielhaften Aufbau einer Vorrichtung in Form einer Laserbearbeitungsmaschine 1, die einen Bearbeitungskopf 3 zur Fokussierung eines Laserstrahls 2 auf ein von dem Bearbeitungskopf 3 beabstandetes Werkstück 4 umfasst. Der Laserstrahl 2 wird im gezeigten Beispiel von einem CO₂-Laser erzeugt. Alternativ kann der Laserstrahl 2 beispielsweise durch einen Festkörperlaser erzeugt werden. Der Laserstrahl 2 wird zur Durchführung einer Werkstückbearbeitung an dem Werkstück 4, beispielsweise in Form eines Laserschweiß- oder Laserschneidprozesses, mittels einer Fokussiereinrichtung in Form einer Fokussierlinse 5 auf das Werkstück 4 fokussiert.

Der Bearbeitungskopf 3 umfasst des Weiteren eine Bearbeitungsdüse 6, wobei die Fokussierlinse 5 im gezeigten Beispiel den Laserstrahl 2 durch die Bearbeitungsdüse 6, genauer gesagt durch eine Öffnung 7 an der Innenseite der Bearbeitungsdüse 6, auf das Werkstück 4 fokussiert, und zwar auf eine an der Oberseite des Werkstücks 4 gebildete Werkstückoberfläche 8, an welcher der Laserstrahl 2 im gezeigten Beispiel an einer Fokusposition F auftrifft.

In Fig. 1 ebenfalls zu erkennen ist ein teildurchlässiger Spiegel 10, durch den der von einer Strahlführung einfallende Laserstrahl 2 hindurch tritt und auf die Fokussierlinse 5 trifft. An dem teildurchlässigen Spiegel 10 wird Beobachtungsstrahlung (beispielsweise im sichtbaren Wellenlängenbereich) eines in Fig. 1 gestrichelt dargestellten Beobachtungsstrahlengangs 12 umgelenkt und gelangt über eine weitere Linse 9 sowie einen weiteren teildurchlässigen Spiegel 14 auf eine

Bilderfassungseinrichtung 13 in Form einer Kamera. Bei der Bilderfassungseinrichtung 13 kann es sich um eine hochauflösende Kamera handeln, die insbesondere als Hochgeschwindigkeitskamera ausgebildet sein kann. Im dargestellten Beispiel erfolgt die Aufnahme von Bildern durch die Bilderfassungseinrichtung 13 im NIR-Wellenlängenbereich. Auch eine Aufnahme von Bildern im VIS- oder UV-Bereich ist möglich. Bei dem in Fig. 1 gezeigten Beispiel kann ein Filter vor der Bilderfassungseinrichtung 13 angeordnet werden, wenn weitere Strahlungs- bzw. Wellenlängenanteile von der Erfassung mit der Bilderfassungseinrichtung 13 ausgeschlossen werden sollen. Der Filter kann z.B. als schmalbandiger Bandpassfilter ausgebildet sein.

Die weitere Linse 9 dient gemeinsam mit der Fokussierlinse 5 als Abbildungsoptik zur Abbildung der Werkstückoberfläche 8 auf eine Detektorfläche 13a der Kamera 13. Die Abbildungsoptik bzw. die Kamera 13 sind so angeordnet, dass der Beobachtungsstrahlengang 12 koaxial zur in Fig. 1 strichpunktiert dargestellten Laserstrahlachse 19 bzw. deren Verlängerung verläuft. Da die Laserstrahlachse 19 mit der Hauptachse 5a der Fokussierlinse 5 übereinstimmt, erfolgt die Beobachtung der Werkstückoberfläche 8 bzw. die Aufnahme des Bildes der Werkstückoberfläche 8 in einer Beobachtungsrichtung R koaxial zur Hauptachse 5a der Fokussierlinse 5 sowie zur Längsachse der typischer Weise rotationssymmetrischen Bearbeitungsdüse 6 und somit richtungsunabhängig.

Der Bearbeitungskopf 3 umfasst weiterhin eine Beleuchtungseinrichtung 15, die der Beleuchtung der von dem Bearbeitungskopf 3 beabstandeten Oberfläche 8 des Werkstücks 4 dient. Die Beleuchtungseinrichtung 15 weist eine Beleuchtungsquelle 16 auf, die einen in Fig. 1 gestrichelt dargestellten Beleuchtungsstrahl 17 erzeugt. Als Beleuchtungsquelle 16 kann insbesondere ein Diodenlaser oder ggf. eine LED vorgesehen werden, z.B. bei einer Wellenlänge λ_{B} von 660nm, 808nm, 915nm oder 980nm. Der Beleuchtungsstrahl 17 tritt durch den weiteren teildurchlässigen Spiegel 14 sowie die weitere Linse 9 hindurch und wird an dem teildurchlässigen Spiegel 10 in Richtung auf die Fokussierlinse 5 umgelenkt, wobei der Beleuchtungsstrahl 17 koaxial zum Laserstrahl 2 bzw. zur Hauptachse 5a der Fokussierlinse 5 ausgerichtet wird, so dass dieser durch die Öffnung 7 der Bearbeitungsdüse 6 tritt und die Oberfläche 8 des Werkstücks 4 beleuchtet.

Nachfolgend wird ein Verfahren beschrieben, wie mit Hilfe der in Fig. 1 gezeigten Laserbearbeitungsmaschine 1 die Werkstoffart und/oder die Oberflächenbeschaffenheit der Oberfläche 8 des Werkstücks 4 bestimmt werden können. Es versteht sich, dass die Bestimmung der Werkstoffart und/oder der Oberflächenbeschaffenheit des Werkstücks 4 nicht zwingend während der in Fig. 1 gezeigten Werkstückbearbeitung erfolgen muss, sondern dass diese insbesondere auch kurz vor dem Beginn der Werkstückbearbeitung erfolgen kann.

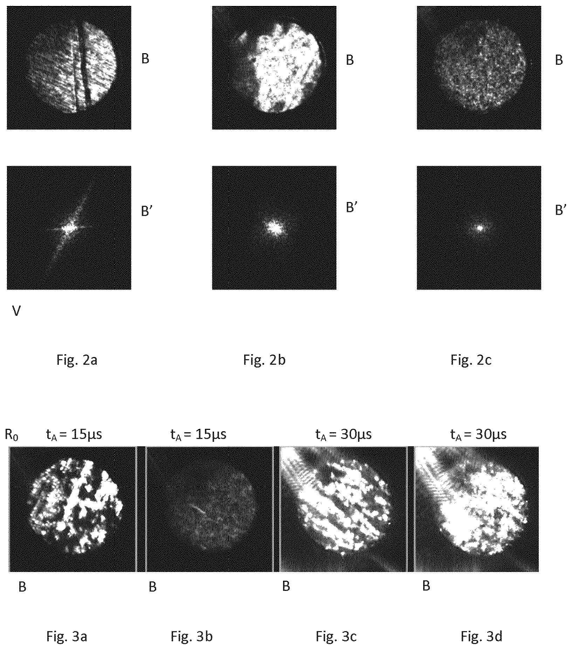

In **Fig. 2a****-c** oben ist jeweils ein von der Kamera 13 aufgenommenes Bild B der Oberfläche 8 eines Werkstücks 4 dargestellt. Eine kreisförmige Randkontur der Bearbeitungsdüse 6 bildet eine Begrenzung des Beobachtungsbereichs, durch den die Oberfläche 8 des Werkstücks 4 beobachtet werden kann. Der durch die Öffnung 7 hindurch erkennbare Ausschnitt der Werkstückoberfläche 8 weist eine jeweils unterschiedliche, charakteristische Oberflächenbeschaffenheit auf.

Bei dem Werkstück 4, dessen Oberfläche 8 in den in Fig. 2a-c gezeigten Bildern B dargestellt ist, handelt es sich in allen drei Fällen um ein gewalztes Edelstahlblech. In Fig. 2a handelt es sich bei dem Werkstück 4 um ein blankes Edelstahlblech, d.h. das Bild B ist eine Abbildung der blanken Oberfläche 8 des Edelstahlblechs. Bei dem in Fig. 2b gezeigten Bild B ist das Edelstahlblech mit einer nicht schneidfähigen Folie beschichtet und bei dem in Fig. 2c gezeigten Bild B ist das Edelstahlblech mit einer schneidfähigen Folie beschichtet.

Um anhand der in Fig. 2a-c gezeigten Bilder B die unterschiedlichen Oberflächenbeschaffenheiten (blanke Oberfläche bzw. unterschiedliche Beschichtungen) besser unterscheiden zu können, werden die in Fig. 2a-c gezeigten Bilder B der Oberfläche 8 des Werkstücks 4 in den Orts-Frequenzraum umgewandelt (d.h. Fourier-transformiert), wobei sich die in Fig. 2a-c unten dargestellten Bilder B' im Orts-Frequenzraum ergeben. In dem in Fig. 2a gezeigten, in den Orts-Frequenzraum gewandelten Bild B' ist deutlich eine Anisotropie der Orts-Frequenzen bzw. ein richtungsabhängiger Orts-Frequenzraum mit einer gestrichelt angedeuteten Vorzugsrichtung V zu erkennen, die auf die Riefenstruktur des entlang einer Walzrichtung gewalzten Edelstahlblechs zurückzuführen ist. Die in Fig. 2b und Fig. 2c dargestellten, in den Orts-Frequenzraum gewandelten Bilder B' sind im Orts-Frequenzraum im Wesentlichen isotrop, unterscheiden sich aber in Streuung der Werte der Häufigkeits-Verteilung der Orts-Frequenzen, d.h. das in Fig. 2b gezeigte, in den Orts-Frequenzraum transformierte Bild B' weist eine stärkere Streuung der Ortsfrequenzen auf, während das in Fig. 2c gezeigte, in den Orts-Frequenzraum transformierte Bild B' stärker um das Zentrum bei niedrigen Orts-Frequenzen konzentriert ist. Die Unterschiede zwischen den in Fig. 2b und in Fig. 2c dargestellten Häufigkeits-Verteilungen können gut durch ein richtungsunabhängiges (skalares) Streuungsmaß für Häufigkeits-Verteilungen beschrieben werden, beispielsweise durch die mittlere absolute Abweichung der einzelnen Werte der Häufigkeits-Verteilung vom Mittelwert der Häufigkeits-Verteilung oder durch die (richtungsunabhängige) Standard-Abweichung bzw. Varianz.

Die Oberflächenbeschaffenheit der Werkstücke 4 lässt sich somit anhand der drei in Fig. 2a-c gezeigten, in den Orts-Frequenzraum gewandelten Bilder B' bestimmen, indem ein Streuungsmaß, welches die Anisotropie der Orts-Frequenzen der Häufigkeits-Verteilung und/oder mindestens ein richtungsunabhängiges Streuungsmaß der Häufigkeits-Verteilung der Orts-Frequenzen berechnet werden. Eine solche Berechnung des Streuungsmaßes kann mit Hilfe von aus der Bildverarbeitung bekannten Methoden, beispielsweise durch Berechnung der örtlichen Momente des in den Orts-Frequenzraum gewandelten Bildes B' erfolgen. Die örtlichen Momente bilden ein zweidimensionales Streuungsmaß, aus dem sich die Anisotropie der Häufigkeits-Verteilung ergibt. Für eine derartige Berechnung ist in der Laserbearbeitungsmaschine 1 eine Auswerteeinrichtung 20 vorgesehen, die mit der Kamera 13 in signaltechnischer Verbindung steht. Die Auswerteeinrichtung 20 kann in die Laserbearbeitungsmaschine 1 beispielsweise in Form eines PCs oder dergleichen integriert sein sowie mit einer Steuer- bzw. Regeleinrichtung 21 in signaltechnischer Verbindung stehen.

Um anhand der Anisotropie der Häufigkeits-Verteilung der Orts-Frequenzen und/oder anhand mindestens einer richtungsunabhängigen Eigenschaft, typischer Weise eines Streuungsmaßes, der Häufigkeits-Verteilung der Orts-Frequenzen eines jeweiligen in den Orts-Frequenzraum gewandelten Bildes B' die Oberflächenbeschaffenheit des Werkstücks 4 zu bestimmen, ist die Auswerteeinrichtung 20 ausgebildet, die bei der Analyse des jeweiligen Bildes B' erhaltenen Werte mit Referenzdaten bzw. Referenzwerten für unterschiedliche Oberflächenbeschaffenheiten zu vergleichen, die typischer Weise in einer Datenbank hinterlegt sind, auf welche die Auswerteeinrichtung 20 Zugriff hat. Für das Vergleichen kann in der Auswerteeinrichtung 20 ein lernendes System, beispielsweise in Form eines künstlichen Neuronalen Netzes, implementiert sein. An Stelle eines lernenden Systems kann die Auswerteeinrichtung 20 andere Verfahren für den Vergleich verwenden, beispielsweise das so genannte "Template Matching", bei dem kleine Teilbereiche eines Bildes mit vorgegebenen Bildbestandteilen ("Templates") verglichen werden, SAD, etc. Es ist aber auch möglich, dass die Auswerteeinrichtung 20 den Vergleich bzw. die Suche ähnlicher Parameter in der Datenbank mit Hilfe einer herkömmlichen Minimierungsfunktion durchführt, beispielsweise durch die Minimierung der kleinsten Fehlerquadrate.

Zusätzlich zur Unterscheidung bzw. zur Klassifizierung von Werkstücken 4 hinsichtlich ihrer Oberflächenbeschaffenheit kann die Auswerteeinrichtung 20 auch unterschiedliche Werkstoffarten bestimmen. Zu diesem Zweck können Referenzdaten bzw. Referenzwerte für unterschiedliche Werkstoffarten in der Datenbank hinterlegt werden und ebenfalls mit der jeweils bei der Analyse bestimmten Werten für die skalaren oder richtungsabhängigen Eigenschaften der Häufigkeits-Verteilung der Orts-Frequenzen verglichen werden.

Zusätzlich zu den beiden weiter oben beschriebenen Unterscheidungskriterien für unterschiedliche Werkstoffe und/oder unterschiedliche Oberflächenbeschaffenheiten kann als weiteres Unterscheidungskriterium der Reflexionsgrad der Oberfläche 8 des Werkstücks 4 herangezogen werden. Zu diesem Zweck kann beispielsweise die Belichtungszeit t_{A} (vgl. Fig. 3a-d), mit der die Kamera 13 ein jeweiliges Bild B aufnimmt, und/oder die Beleuchtungsintensität I (vgl. Fig. 1) angepasst werden, bis das Bild B einen ausreichenden Kontrast aufweist.

Anhand der angepassten Belichtungszeit t_{A} und der angepassten Beleuchtungsintensität I kann auf den Reflexionsgrad der Oberfläche 8 des Werkstücks 4 geschlossen werden, der ein erstes Indiz für die Werkstoffart und/oder die Oberflächenbeschaffenheit des Werkstücks 4 liefert. Der Reflexionsgrad kann auch bei mehreren Wellenlängen der Beleuchtungsstrahlung 17 bestimmt werden, um die Klassifizierung unterschiedlicher Werkstoffarten bzw. Oberflächenbeschaffenheiten zu verbessern. In diesem Fall können die für unterschiedliche Wellenlängen der Beleuchtungsstrahlung 17 bestimmten Reflexionsgrade miteinander in Beziehung gesetzt werden (relative Reflexion), um die Oberflächenbeschaffenheit und/oder die Werkstoffart zu bestimmen.

**Fig. 3a**-**d** zeigen vier mit der Kamera 13 aufgenommene Bilder B vonunterschiedlichen Baustahlsorten, wobei die in Fig. 3a,b gezeigten Bilder B mit einer Belichtungszeit t_{A} von 15 µs und die in Fig. 3c,d gezeigten Bilder B mit einer Belichtungszeit t_{A} von 30 µs aufgenommen wurden. Eine Unterscheidung zwischen den Baustahlsorten ist durch den unterschiedlichen Reflexionsgrad R_{O} sowie anhand der Riefenstruktur möglich, die anhand der in Fig. 3a-d nicht dargestellten, in den Orts-Frequenzraum umgewandelten Bilder der Oberfläche 8 des Werkstücks 4 analysiert werden kann.

Auf die oben beschriebene Weise können insbesondere die Werkstoffarten Baustahl, Edelstahl und Buntmetalle voneinander unterschieden werden, wobei auch unterschiedliche Buntmetalle und unterschiedliche Baustahlsorten unterschieden werden können. Anders als im Zusammenhang mit Fig. 2a-c und Fig. 3a-d beschrieben wurde, ist es günstig, an Stelle eines einzelnen in den Orts-Frequenzraum gewandelten Bildes einen (pixelweisen) Mittelwert aus einer Mehrzahl von zeitlich aufeinander folgend aufgenommenen Bildern, z.B. zwischen 10 und 1000 in den Orts-Frequenzbereich gewandelten Bildern B' zu bilden und die Bestimmung der Werkstoffart und/oder der Oberflächenbeschaffenheit anhand dieses Mittelwerts vorzunehmen. Der Mittelwert wird in diesem Fall zum Vergleich mit den jeweiligen Referenzdaten herangezogen. Es kann ggf. günstig sein, auch für die Bestimmung des Reflexionsgrades einen Mittelwert aus einer Mehrzahl von zeitlich aufeinander folgend aufgenommenen Bildern zu bestimmen.

Abhängig von der auf die oben beschriebene Weise bestimmten Werkstoffart und/oder Oberflächenbeschaffenheit können Bearbeitungsparameter des Bearbeitungsprozesses, im vorliegenden Beispiel eines Laserschweißprozesses oder eines Laserschneidprozesses, z.B. die Vorschubgeschwindigkeit, die Laserleistung, die Art des dem Werkstück als Hilfsgas oder als Schneidgas zugeführten Gases sowie dessen Gasdruck geeignet gewählt werden. Die Auswahl der Bearbeitungsparameter kann automatisiert in der Steuer- und/oder Regeleinrichtung 21 erfolgen, die zu diesem Zweck auf eine Datenbank zurückgreifen kann. Auch kann in der Steuer- und/oder Regeleinrichtung 21 ggf. eine Auswahl von geeigneten Bearbeitungstechnologien, beispielsweise die Auswahl einer für die Bearbeitung geeigneten Laserquelle erfolgen, wenn in der Laserbearbeitungsmaschine 1 zwischen mehreren Laserquellen zur Erzeugung des Laserstrahls 2 ausgewählt werden kann.

Durch die automatisierte Erkennung bzw. Bestimmung der Werkstoffart und der Oberflächenbeschaffenheit können Fehlerquellen bei der manuellen Eingabe der Materialart durch einen Bediener vermieden werden. Auch kann eine Zerstörung der Bearbeitungsmaschine durch Rückreflexion des Laserstrahls 2 an hochreflektierenden Werkstoffen vermieden werden. Auch kann eine automatische Optimierung der Bearbeitungsparameter erfolgen, wobei beispielsweise eine automatische Vorbehandlung folierter Werkstücke vorgenommen werden kann. Auch können die Beleuchtungseinrichtung 15 und die Bilderfassungseinrichtung 13 in vorteilhafter Weise für die Messung bzw. Bestimmung anderer für den Bearbeitungsprozess relevanter Messgrößen verwendet werden.

## Patentansprüche

1. Verfahren zum Bestimmen einer Werkstoffart und/oder einer Oberflächenbeschaffenheit eines bevorzugt metallischen, insbesondere plattenförmigen Werkstücks (4), umfassend die Schritte:
Beleuchten einer Oberfläche (8) des Werkstücks (4) mit Beleuchtungsstrahlung (17), bevorzugt mit Laserstrahlung als Beleuchtungsstrahlung (17), Aufnehmen mindestens eines Bildes (B) der beleuchteten Oberfläche (8), sowie Bestimmen der Werkstoffart und/oder der Oberflächenbeschaffenheit des Werkstücks (4) anhand einer statistischen Analyse des mindestens einen in den Orts-Frequenzraum gewandelten Bildes (B'), **dadurch gekennzeichnet, dass** die Werkstoffart und/oder die Oberflächenbeschaffenheit des Werkstücks (4) anhand einer Anisotropie der Häufigkeits-Verteilung der Orts-Frequenzen und/oder anhand mindestens einer richtungsunabhängigen Eigenschaft der Häufigkeits-Verteilung der Orts-Frequenzen des in den Orts-Frequenzraum gewandelten Bildes (B') bestimmt werden.

2. Verfahren nach Anspruch 1, bei dem beim Bestimmen der Werkstoffart und/oder der Oberflächenbeschaffenheit des Werkstücks (4) zusätzlich der Reflexionsgrad (R_{O}) der Oberfläche (8) des Werkstücks (4) berücksichtigt wird.

3. Verfahren nach Anspruch 2, bei welchem zum Bestimmen der Werkstoffart und/oder der Oberflächenbeschaffenheit der Reflexionsgrad (R_{O}), die Anisotropie und/oder die Häufigkeits-Verteilung der Orts-Frequenzen mit Referenzdaten für unterschiedliche Werkstoffarten und/oder Oberflächenbeschaffenheiten verglichen werden, wobei das Vergleichen bevorzugt mittels eines lernenden Systems erfolgt.

4. Verfahren nach einem der vorhergehenden Ansprüche, bei welchem die Werkstoffart bestimmt wird aus einer Gruppe umfassend: Baustahl, Edelstahl und Buntmetalle.

5. Verfahren nach einem der vorhergehenden Ansprüche, bei welchem das Bestimmen der Oberflächenbeschaffenheit das Erkennen einer gewalzten Oberfläche (8) des Werkstücks (4) umfasst.

6. Verfahren nach einem der vorhergehenden Ansprüche, bei welchem beim Aufnehmen des Bildes (B) eine Belichtungszeit (t_{A}) und/oder eine Beleuchtungsintensität (I) an den Reflexionsgrad (R_{O}) der Oberfläche (8) des Werkstücks (4) angepasst werden.

7. Verfahren nach einem der vorhergehenden Ansprüche, bei welchem das Bestimmen der Werkstoffart und/oder der Oberflächenbeschaffenheit das Bilden eines Mittelwerts aus einer Zahl zwischen 10 und 1000 in den Orts-Frequenzraum gewandelter Bilder (B') umfasst.

8. Verfahren nach einem der vorhergehenden Ansprüche, bei welchem die Beleuchtungsstrahlung (17) koaxial zu einem Hochenergiestrahl (2) zur Bearbeitung des Werkstücks (4) auf die Oberfläche (8) eingestrahlt wird.

9. Verfahren nach einem der vorhergehenden Ansprüche, weiter umfassend: Festlegen mindestens eines Bearbeitungsparameters zum Bearbeiten des Werkstücks (4) abhängig von der Werkstoffart und/oder der Oberflächenbeschaffenheit.

10. Computerprogrammprodukt, welches Codemittel aufweist, die zur Durchführung aller Schritte des Verfahrens nach einem der vorhergehenden Ansprüche angepasst sind, wenn das Computerprogramm auf einer Datenverarbeitungsanlage abläuft.

11. Vorrichtung (1) zur Bestimmung einer Werkstoffart und/oder einer Oberflächenbeschaffenheit eines bevorzugt metallischen, insbesondere plattenförmigen Werkstücks (4), umfassend:
eine Beleuchtungseinrichtung (15) zur Erzeugung von Beleuchtungsstrahlung (17) zum Beleuchten einer Oberfläche (8) des Werkstücks (4), wobei die Beleuchtungseinrichtung (15) bevorzugt als Beleuchtungsquelle mindestens einen Laser, insbesondere einen Diodenlaser (16), oder eine Leuchtdiode umfasst,
eine Bilderfassungseinrichtung (13) zur Aufnahme mindestens eines Bildes (B) der beleuchteten Oberfläche (8) des Werkstücks (4), sowie eine Auswerteeinrichtung (20) zur Bestimmung der Werkstoffart und/oder der Oberflächenbeschaffenheit des Werkstücks (4) anhand einer statistischen Analyse des mindestens einen in den Orts-Frequenzraum gewandelten Bildes (B'), **dadurch gekennzeichnet, dass** die Auswerteeinrichtung (20) ausgebildet ist, die Werkstoffart und/oder die Oberflächenbeschaffenheit des Werkstücks (4) anhand einer Anisotropie der Häufigkeits-Verteilung der Orts-Frequenzen und/oder anhand mindestens einer richtungsunabhängigen Eigenschaft der Häufigkeits-Verteilung der Orts-Frequenzen des in den Orts-Frequenzraum gewandelten Bildes (B') zu bestimmen.

12. Vorrichtung nach Anspruch 11, weiter umfassend: eine Fokussierlinse (5) zum Fokussieren eines Hochenergiestrahls (2) auf das Werkstück (4), wobei die Bilderfassungseinrichtung (13) zur Aufnahme des mindestens einen Bildes (B) mittels eines durch die Fokussierlinse (5) verlaufenden Beobachtungsstrahlengangs (12) ausgebildet ist.

13. Vorrichtung nach Anspruch 12, bei welcher die Bilderfassungseinrichtung (13) ausgebildet ist, das mindestens eine Bild (B) aus einer Beobachtungsrichtung (R) koaxial zur Hauptachse (5a) der Fokussierlinse (5) aufzunehmen.

14. Vorrichtung nach einem der Ansprüche 12 oder 13, bei welcher die Beleuchtungseinrichtung (15) zur Beleuchtung der Oberfläche (8) des Werkstücks (4) durch die Fokussierlinse (5), bevorzugt koaxial zur Hauptachse (5a) der Fokussierlinse (5), ausgebildet ist.

15. Vorrichtung nach einem der Ansprüche 11 bis 14, bei welcher die Auswerteeinrichtung (20) ausgebildet ist, zum Bestimmen der Werkstoffart und/oder der Oberflächenbeschaffenheit des Werkstücks (4) zusätzlich den Reflexionsgrad (R_{O}) der Oberfläche (8) des Werkstücks (4) zu berücksichtigen.

16. Vorrichtung nach Anspruch 15, bei welcher die Auswerteeinrichtung (20) ausgebildet ist, zum Bestimmen der Werkstoffart und/oder der Oberflächenbeschaffenheit den Reflexionsgrad (R_{O}), die Anisotropie der Orts-Frequenzen und/oder die Häufigkeits-Verteilung der Orts-Frequenzen mit Referenzdaten für unterschiedliche Werkstoffarten und/oder Oberflächenbeschaffenheiten zu vergleichen.

## Claims

1. A method for determining a material type and/or a surface condition of a preferably metallic, in particular plate-shaped, workpiece (4), comprising the steps:
illuminating a surface (8) of the workpiece (4) with illuminating radiation (17), preferably using laser radiation as illuminating radiation (17),
recording at least one image (B) of the illuminated surface (8),
and determining the material type and/or the surface condition of the workpiece (4) on the basis of a statistical analysis of the at least one image (B') converted into the spatial frequency domain,
**characterized in that**
the material type and/or surface condition of the workpiece (4) are determined on the basis of an anisotropy of the frequency distribution of the spatial frequencies and/or on the basis of at least one direction-independent property of the frequency distribution of the spatial frequencies of the image (B') converted into the spatial frequency domain.

2. The method as claimed in claim 1, wherein the reflectance (R_{O}) of the surface (8) of the workpiece (4) is additionally taken into account during the determination of the material type and/or surface condition of the workpiece (4).

3. The method as claimed in claim 2, wherein the reflectance (R_{O}), the anisotropy and/or the frequency distribution of the spatial frequencies are compared with reference data for different material types and/or surface conditions in order to determine the material type and/or the surface condition, wherein the comparison is preferably carried out with the aid of a learning system.

4. The method as claimed in one of the preceding claims, wherein the material type is determined from a group comprising: construction steel, stainless steel and nonferrous metals.

5. The method as claimed in one of the preceding claims, wherein the determination of the surface condition comprises the detection of a rolled surface (8) of the workpiece (4).

6. The method as claimed in one of the preceding claims, wherein an exposure time (t_{A}) and/or an illumination intensity (I) is/are adapted to the reflectance (R_{O}) of the surface (8) of the workpiece (4) during the recording of the image (B).

7. The method as claimed in one of the preceding claims, wherein the determination of the material type and/or the surface condition comprises the formation of an average value from a number of between 10 and 1000 images (B') converted into the spatial frequency domain.

8. The method as claimed in one of the preceding claims, wherein the illuminating radiation (17) is irradiated onto the surface (8) coaxially with a high-energy beam (2) for processing the workpiece (4).

9. The method as claimed in one of the preceding claims, further comprising: determining at least one processing parameter for the processing of the workpiece (4) as a function of the material type and/or the surface condition.

10. A computer program product which comprises code means that are adapted to carry out all steps of the method as claimed in one of the preceding claims when the computer program runs on a data processing unit.

11. An apparatus (1) for determining a material type and/or a surface condition of a preferably metallic, in particular plate-shaped, workpiece (4), comprising:
an illuminating device (15) for generating illuminating radiation (17) for illuminating a surface (8) of the workpiece (4), wherein the illuminating device (15) preferably comprises at least one laser, in particular a diode laser (16), or a light-emitting diode, as the illumination source,
an image acquisition device (13) for recording at least one image (B) of the illuminated surface (8) of the workpiece (4), and
an evaluation device (20) for determining the material type and/or the surface condition of the workpiece (4) on the basis of a statistical analysis of the at least one image (B') converted into the spatial frequency domain,
**characterized in that**
the evaluation device (20) is configured to determine the material type and/or the surface condition of the workpiece (4) on the basis of an anisotropy of the frequency distribution of the spatial frequencies and/or on the basis of at least one direction-independent property of the frequency distribution of the spatial frequencies of the image (B') converted into the spatial frequency domain.

12. The apparatus as claimed in claim 11, further comprising a focusing lens (5) for focusing a high-energy beam (2) onto the workpiece (4), wherein the image acquisition device (13) is configured to record the at least one image (B) by means of an observation beam path (12) extending through the focusing lens (5).

13. The apparatus as claimed in claim 12, wherein the image acquisition device (13) is configured to record the at least one image (B) from an observation direction (R) coaxial with the principal axis (5a) of the focusing lens (5).

14. The apparatus as claimed in one of claims 12 or 13, wherein the illuminating device (15) is configured to illuminate the surface (8) of the workpiece (4) through the focusing lens (5), preferably coaxially with the principal axis (5a) of the focusing lens (5).

15. The apparatus as claimed in one of claims 11 to 14, wherein the evaluation device (20) is configured additionally to take the reflectance (R_{O}) of the surface (8) of the workpiece (4) into account in order to determine the material type and/or surface condition of the workpiece (4).

16. The apparatus as claimed in claim 15, wherein the evaluation device (20) is configured to compare the reflectance (R_{O}), the anisotropy of the spatial frequencies and/or the frequency distribution of the spatial frequencies with reference data for different material types and/or surface conditions in order to determine the material type and/or the surface condition.

## Revendications

1. Procédé pour déterminer un type de matériau et/ou un état de surface d'une pièce de préférence métallique, en particulier en forme de plaque (4), comprenant les étapes suivantes :
éclairage d'une surface (8) de la pièce (4) avec un rayonnement lumineux (17), de préférence un rayonnement laser comme rayonnement lumineux (17),
capture d'au moins une image (B) de la surface éclairée (8) et détermination du type de matériau et/ou de l'état de surface de la pièce (4) à l'aide d'une analyse statistique de ladite au moins une image (B') transformée dans le domaine spatial - fréquentiel,
**caractérisé en ce que** le type de matériau et/ou l'état de surface de la pièce (4) sont déterminés à l'aide d'une anisotropie de la distribution de fréquence des fréquences spatiales et/ou à l'aide d'au moins une propriété indépendante de la direction de la distribution de fréquence des fréquences spatiales de l'image (B') transformée dans le domaine spatial - fréquentiel.

2. Procédé selon la revendication 1, dans lequel le degré de réflexion (Ro) de la surface (8) de la pièce (4) est en outre pris en compte pour déterminer le type de matériau et/ou l'état de surface de la pièce (4).

3. Procédé selon la revendication 2, dans lequel, pour déterminer le type de matériau et/ou l'état de surface, le degré de réflexion (R_{O}), l'anisotropie et/ou la distribution de fréquence des fréquences spatiales sont comparés avec des données de référence pour différents types de matériaux et/ou états de surface, la comparaison étant effectuée de préférence au moyen d'un système d'apprentissage.

4. Procédé selon l'une des revendications précédentes dans lequel le type de matériau est déterminé dans un groupe comprenant : acier de construction, acier inoxydable et métaux non ferreux.

5. Procédé selon l'une des revendications précédentes dans lequel la détermination de l'état de surface comprend la reconnaissance d'une surface laminée (8) de la pièce (4).

6. Procédé selon l'une des revendications précédentes dans lequel, lors de la capture de l'image (B), un temps d'exposition (t_{A}) et/ou une intensité d'éclairage (I) sont adaptés au degré de réflexion (R_{O}) de la surface (8) de la pièce (4).

7. Procédé selon l'une des revendications précédentes dans lequel la détermination du type de matériau et/ou de l'état de surface comprend la formation d'une moyenne à partir d'un nombre compris entre 10 et 1000 dans les images (B') transformées dans le domaine spatial - fréquentiel.

8. Procédé selon l'une des revendications précédentes, dans lequel le rayonnement d'éclairage (17) est projeté sur la surface (8) coaxialement à un faisceau à haute énergie (2) servant à usiner la pièce (4).

9. Procédé selon l'une des revendications précédentes, comprenant en outre :
la détermination d'au moins un paramètre d'usinage pour usiner la pièce (4) en fonction du type de matériau et/ou de l'état de surface.

10. Produit programme d'ordinateur présentant des moyens de code qui sont adaptés à mettre en oeuvre toutes les étapes du procédé selon l'une des revendications précédentes lorsque le programme d'ordinateur s'exécute sur un système de traitement de données.

11. Dispositif (1) pour déterminer un type de matériau et/ou un état de surface d'une pièce de préférence métallique, en particulier en forme de plaque (4), comprenant :
un moyen d'éclairage (15) pour produire un rayonnement d'éclairage (17) pour éclairer une surface (8) de la pièce (4), le moyen d'éclairage (15) comprenant de préférence au moins un laser, en particulier un laser à diode (16), ou une diode électroluminescente comme source d'éclairage,
un moyen de capture d'image (13) pour capturer au moins une image (B) de la surface éclairée (8) de la pièce (4) et
un moyen d'évaluation (20) pour déterminer le type de matériau et/ou l'état de surface de la pièce (4) à l'aide d'une analyse statistique de ladite au moins une image (B') transformée dans le domaine spatial - fréquentiel,
**caractérisé en ce que** le moyen d'évaluation (20) est conçu pour déterminer le type de matériau et/ou l'état de surface de la pièce (4) à l'aide d'une anisotropie de la distribution de fréquence des fréquences spatiales et/ou à l'aide d'au moins une propriété indépendante de la direction de la distribution de fréquence des fréquences spatiales de l'image (B') transformée dans le domaine spatial - fréquentiel.

12. Dispositif selon la revendication 11, comprenant en outre: une lentille de focalisation (5) pour focaliser un faisceau à haute énergie (2) sur la pièce (4), le moyen de capture d'image (13) étant conçu pour capturer ladite au moins une image (B) au moyen d'un trajet de faisceau d'observation (12) s'étendant à travers la lentille de focalisation (5).

13. Dispositif selon la revendication 12, dans lequel le moyen de capture d'image (13) est conçu pour capturer ladite au moins une image (B) depuis une direction d'observation (R) coaxiale à l'axe principal (5a) de la lentille de focalisation (5).

14. Dispositif selon l'une des revendications 12 ou 13, dans lequel le moyen d'éclairage (15) est conçu pour éclairer la surface (8) de la pièce (4) à travers la lentille de focalisation (5), de préférence coaxialement à l'axe principal (5a) de la lentille de focalisation (5).

15. Dispositif selon l'une des revendications 11 à 14, dans lequel le moyen d'évaluation (20) est conçu pour tenir compte en outre du degré de réflexion (R_{O}) de la surface (8) de la pièce (4) pour déterminer le type de matériau et/ou l'état de surface de la pièce (4).

16. Dispositif selon la revendication 15, dans lequel, pour déterminer le type de matériau et/ou l'état de surface, le moyen d'évaluation (20) est conçu pour comparer le degré de réflexion (R_{O}), l'anisotropie des fréquences spatiales et/ou la distribution de fréquence des fréquences spatiales avec des données de référence pour différents types de matériaux et/ou états de surface.
